# EUROPÄISCHE PATENTSCHRIFT

(11) **EP 3 676 258 B1**
(45) Veröffentlichungstag und Bekanntmachung des Hinweises auf die Patenterteilung: **05.10.2022**
(21) Anmeldenummer: 18756431.5
(22) Anmeldetag: 20.08.2018
(51) Int. Cl.: C11D 1/14, C11D 1/29, C07D 307/10

(54) **NEUE ANIONISCHE TENSIDE UND WASCH- UND REINIGUNGSMITTEL, WELCHE DIESE ENTHALTEN**
NEW ANIONIC SURFACTANTS AND DETERGENTS AND CLEANING AGENTS CONTAINING SAME
NOUVEAUX TENSIOACTIFS ANIONIQUES ET PRODUITS DÉTERGENTS ET NETTOYANTS LES CONTENANT

(30) Priorität: 28.08.2017 DE 102017008073
(43) Veröffentlichungstag der Anmeldung: 08.07.2020
(73) Patentinhaber: Henkel AG & Co. KGaA, 40589 Düsseldorf (DE)
(72) Erfinder: KROPF, Christian, 40724 Hilden (DE); KLEMMER, Anna, 40597 Düsseldorf (DE); BEDRUNKA, Danuta, 41541 Dormagen (DE); PALKOVITS, Regina, 52074 Aachen (DE); HAUSOUL, Peter, 6371 GE Landgraaf (NL); KIPSHAGEN, Lukas, 52064 Aachen (DE)
(86) Internationale Anmeldenummer: PCT/EP2018/072409
(87) Internationale Veröffentlichungsnummer: WO 2019/042805

(56) Entgegenhaltungen:
- US-A1- 2015 150 768

## Beschreibung

Die Erfindung betrifft anionische Tenside, die auf Basis nachwachsender Rohstoffe hergestellt werden können und die niedrige kritische Mizellbildungskonzentrationen (CMC) aufweisen sowie niedrige Grenzflächenspannungen erzeugen. Die Erfindung betrifft auch ein Verfahren zur Herstellung derartiger Tenside sowie Wasch- oder Reinigungsmittel, welche diese Tenside enthalten.

Der Einsatz von Tensiden zur Herabsetzung der Oberflächenspannung von Wasser, zur Bildung von Dispersionen und zur Lösungsvermittlung ist auf dem Gebiet der Wasch- und Reinigungsmittel schon lange allgemein bekannt. Obwohl viele Tenside ganz oder teilweise auf Basis nachwachsender Rohstoffe hergestellt werden, sind einige leistungsstarke und breit eingesetzte Vertreter nach wie vor petrochemisch basiert. Zudem existiert der ständige Wunsch, Tenside mit herausragenden anwendungstechnischen Eigenschaften bereitzustellen, um eine hohe Leistung auch bei niedrigem Tensideinsatz erzielen zu können.

Aus der Patentanmeldung US 2015/0150768 A1 sind Furan-basierte Tenside bekannt, in denen ein Furanring einen hydrophoben Substituenten in Form einer gegebenenfalls verzweigten Alkylgruppe und einen hydrophilen Substituenten mit einer Sulfo-, Sulfato- oder Oligoethoxy-Endgruppe trägt.

Aufgabe der vorliegenden Erfindung ist es, Tenside zur Verfügung zu stellen, welche vorteilhafte anwendungstechnische Eigenschaften wie eine niedrige CMC und eine niedrige Oberflächenspannung aufweisen und auf Basis nachwachsender Rohstoffe hergestellt werden können. Darüber hinaus sollten die Tenside gut hautverträglich und auch gemeinsam mit anderen Tensiden konfektionierbar sein, damit sie sich insbesondere für den Einsatz in Wasch- und Reinigungsmitteln eignen.

Gegenstand der vorliegenden Erfindung ist in einer ersten Ausführungsform ein anionisches Tensid der allgemeinen Formel (I), 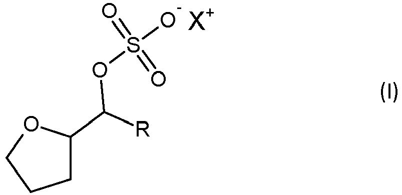 in der R für eine lineare oder verzweigte Alkyl-, Alkenyl-, Alkylaryl- oder Alkenylarylgruppe mit 5 bis 25 C-Atomen und X⁺ für ein ladungsausgleichendes Kation steht. X⁺ wird vorzugsweise ausgewählt aus der Gruppe umfassend das Proton, Alkalimetallkationen und die Gruppierung N⁺R¹R²R³, in der R¹, R² und R³ unabhängig voneinander für Wasserstoff, eine Alkylgruppe mit 1 bis 6 C-Atomen oder eine Hydroxyalkylgruppe mit 2 bis 6 C-Atomen stehen.

Bevorzugte Tenside der allgemeinen Formel (I) sind solche, in denen R für eine lineare oder verzweigte Alkylgruppe mit 7-21 C-Atomen steht, wobei lineare Gruppen R besonders bevorzugt sind.

Tenside der allgemeinen Formel (I) lassen sich durch Sulfatierung einer Verbindung der allgemeinen Formel (II), 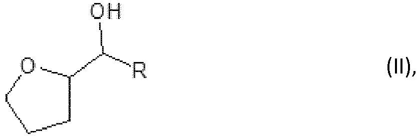 in der R die oben angegebene Bedeutung hat, mit einem Sulfatierungsagens, beispielsweise Chlorsulfonsäure oder Schwefeltrioxid-Pyridin, und gegebenenfalls Neutralisation durch anschließende Umsetzung mit X⁺OH⁻, X⁺HCO⁻₃ oder X⁺₂CO²⁻₃, wobei X⁺ die oben angegebene Bedeutung hat, herstellen. Verbindungen der allgemeinen Formel (II) sind durch Grignard-Reaktion aus Tetrahydrofurfural oder alternativ Furfural und anschließender Hydrierung zugänglich. Bei der Grignard-Reaktion reagiert ein Organo-Magnesiumhalogenid als Nukleophil mit der elektrophilen Carbonylgruppe des Furfurals oder Tetrahydrofurfurals, so dass eine neue Kohlenstoff-Kohlenstoff-Einfachbindung entsteht. Furfural kann aus Cellulose erzeugt werden und fällt beispielsweise als Nebenprodukt bei der Zellstoffherstellung an.

Ein weiterer Gegenstand der Erfindung ist ein Verfahren zur Herstellung einer oben definierten Verbindung der allgemeinen Formel (I) durch a) Umsetzung von Furfural mit einer Grignard-Verbindung R-Mg-Hal, in der R die oben angegebene Bedeutung hat und Hal ausgewählt wird aus Br und J, b) Hydrierung des Furanrings zum Tetrahydrofuranring, und c) Sulfatierung mit einem Sulfatierungsagens und gegebenenfalls Neutralisation durch anschließende Umsetzung mit X⁺OH⁻, X⁺HCO⁻₃ oder X⁺₂CO²⁻₃, wobei X⁺ für ein Alkalimetallkation oder eine Gruppierung N⁺R¹R²R³ steht, in der R¹, R² und R³ unabhängig voneinander für Wasserstoff, eine Alkylgruppe mit 1 bis 6 C-Atomen oder eine Hydroxyalkylgruppe mit 2 bis 6 C-Atomen stehen.

Ein weiterer Gegenstand der Erfindung ist ein alternatives Verfahren zur Herstellung einer oben definierten Verbindung der allgemeinen Formel (I) durch a) Umsetzung von Tetrahydrofurfural mit einer Grignard-Verbindung R-Mg-Hal, in der R die oben angegebene Bedeutung hat und Hal ausgewählt wird aus Br und J, und b) Sulfatierung mit einem Sulfatierungsagens und gegebenenfalls Neutralisation durch anschließende Umsetzung mit X⁺OH⁻, X⁺HCO⁻₃ oder X⁺₂CO²⁻₃, wobei X⁺ für ein Alkalimetallkation oder eine Gruppierung N⁺R¹R²R³ steht, in der R¹, R² und R³ unabhängig voneinander für Wasserstoff, eine Alkylgruppe mit 1 bis 6 C-Atomen oder eine Hydroxyalkylgruppe mit 2 bis 6 C-Atomen stehen.

Die erfindungsgemäßen Tenside weisen sehr niedrige CMC-Werte auf und führen zu sehr niedrigen Grenzflächenspannungen gegenüber Öl mit einer schnellen Dynamik bei der Organisation an der Grenzfläche. Besonders bevorzugte erfindungsgemäße Tenside weisen in Wasser bei pH 8,5 und 25°C eine CMC von 0,005 g/l bis 0,25 g/l auf und erzeugen eine mittels der Spinning Drop Methode (20-minütige Equilibrierungszeit) gegen Isopropylmyristat bestimmbare Grenzflächenspannung bei einer Konzentration von 1 g/l in Wasser bei pH 8,5 und 25 °C von kleiner 8 mN/m, insbesondere im Bereich von 1 mN/m bis 5 mN/m.

Die erfindungsgemäßen Tenside sind wie geschildert aus nachwachsenden Rohstoffen zugänglich. Sie weisen zudem den Vorteil auf, dass unter den nachwachsenden Rohstoffen, aus denen sie hergestellt werden können, solche sind, die keine Basis für die Gewinnung von Nahrungsmitteln darstellen, so dass die bei manchen aus anderen nachwachsenden Rohstoffen erhältlichen Tensiden beobachtete Nahrungsmittelkonkurrenzsituation hier nicht besteht.

Die erfindungsgemäßen Tenside werden vorzugsweise hergestellt, indem man Furfural mit einem aus einer Verbindung R-Hal, in der R und Hal die oben angegebene Bedeutung haben, hergestellten Grignard-Reagenz unter Inertgas bei Temperaturen im Bereich von -5 °C bis 10 °C, insbesondere 0 °C bis 5 °C in einem Zeitraum von vorzugsweise 2 Stunden bis 5 Stunden, insbesondere 3 Stunden bis 5 Stunden, zu Furanylolen umsetzt. Diese werden vorzugsweise bei Temperaturen von 70 °C bis 150 °C, insbesondere 80°C bis 100°C bei einem Wasserstoffdruck von vorzugsweise 80 bar bis 150 bar, insbesondere 120 bar bis 150 bar über einen Zeitraum im Bereich von vorzugsweise 3 Stunden bis 8 Stunden, insbesondere 3 Stunden bis 5 Stunden hydriert. Eine so erhaltene Verbindung wird mit einem Sulfatierungsagens, beispielsweise Chlorsulfonsäure oder Schwefeltrioxid-Pyridin, bei einer Temperatur im Bereich von vorzugsweise -20 °C bis 75 °C, insbesondere von 25 °C bis 75 °C, und einer Dauer im Bereich von vorzugsweise 1 Stunde bis 24 Stunden, insbesondere 6 bis 18 Stunden, umgesetzt. Anschließend kann gewünschtenfalls das nach der Sulfatierung vorhandene ladungsausgleichende Kation durch Umsetzung mit X⁺OH⁻, zum Beispiel 1M methanolischer Natriumhydroxid-Lösung oder durch Umsetzung mit X⁺₂CO²⁻₃, zum Beispiel Natriumcarbonat, ausgetauscht werden. Die Isolierung des Tensides der allgemeinen Formel (I) kann beispielsweise durch Ausfällen bei der Zugabe eines geeigneten Ausfällungsmittels, insbesondere Aceton, Diethylether oder Petrolether, realisiert werden.

Die erfindungsgemäßen Tenside eignen sich hervorragend als Inhaltsstoff in Wasch- und Reinigungsmitteln, Kosmetika wie Shampoos, Zahnpasten, und für die übrigen Einsatzgebiete, in denen üblicherweise bisher anionische Tenside eingesetzt werden, wie zum Beispiel in der Lebensmittelindustrie, den Geowissenschaften, der tertiären Erdölförderung, der Kunststofftechnik, der Metallbearbeitung, der Fotografie, dem Papierrecycling, der Werkzeugreinigung, und der Brandbekämpfung.

Besonders gute Ergebnisse werden bei ihrem Einsatz in Wasch- und Reinigungsmitteln erzielt, so dass weitere Gegenstände der vorliegenden Erfindung die Verwendung von anionischem Tensid der allgemeinen Formel (I) zur Herstellung von Wasch- oder Reinigungsmitteln, die Verwendung eines anionischen Tensids der allgemeinen Formel (I) zur Erhöhung der Leistung von Wasch- oder Reinigungsmitteln beim Waschen von Wäsche oder der Reinigung harter Oberflächen sowie die Wasch- oder Reinigungsmittel sind, die ein Tensid der allgemeinen Formel (I) enthalten.

Ein erfindungsgemäßes Wasch- oder Reinigungsmittel enthält vorzugsweise 1 Gew.-% bis 99 Gew.%, insbesondere 3 Gew.-% bis 65 Gew.-% und besonders bevorzugt 5 Gew.-% bis 45 Gew.-% des Tensids der allgemeinen Formel (I).

Zusätzlich zu dem anionischen Tensid der allgemeinen Formel (I) kann das Wasch- oder Reinigungsmittel weitere Inhaltsstoffe enthalten, welche die anwendungstechnischen und/oder ästhetischen Eigenschaften des Mittels weiter verbessern. Im Rahmen der vorliegenden Erfindung enthält das Mittel vorzugsweise zusätzlich einen oder mehrere Stoffe aus der Gruppe der nichtionische Tenside, anionischen Tenside, Gerüststoffe (Builder), Bleichmittel, Bleichaktivatoren, Enzyme, Elektrolyte, pH-Stellmittel, Parfüme, Parfümträger, Fluoreszenzmittel, Farbstoffe, Hydrotope, Schauminhibitoren, Antiredepositionsmittel, Vergrauungsinhibitoren, Einlaufverhinderer, Knitterschutzmittel, Farbübertragungsinhibitoren, antimikrobiellen Wirkstoffe, nicht-wässrigen Lösungsmittel, Germizide, Fungizide, Antioxidantien, Konservierungsmittel, Korrosionsinhibitoren, Antistatika, Bittermittel, Bügelhilfsmittel, Phobier- und Imprägniermittel, Haut-pflegende Wirkstoffe, Quell- und Schiebefestmittel, weichmachenden Komponenten sowie UV-Absorber.

Ein erfindungsgemäßes Wasch- oder Reinigungsmittel enthält vorzugsweise zusätzlich zu dem anionischen Tensid der allgemeinen Formel (I) bis zu 99 Gew.-%, insbesondere 3 Gew.-% bis 65 Gew.% und besonders bevorzugt 4 Gew.-% bis 45 Gew.-% weiteres Tensid, wobei die zusätzlich vorhandenen Tenside vorzugsweise ebenfalls aus nachwachsenden Rohstoffen erhältlich sind.

Das erfindungsgemäße Mittel kann nichtionische Tenside enthalten. Geeignete nichtionische Tenside umfassen alkoxylierte Fettalkohole, alkoxylierte Fettsäurealkylester, Fettsäureamide, alkoxylierte Fettsäureamide, Polyhydroxyfettsäureamide, Alkylphenolpolyglycolether, Aminoxide, Alkylpolyglucoside und Mischungen daraus.

Als alkoxylierte Fettalkohole werden vorzugsweise ethoxylierte, insbesondere primäre Alkohole mit vorzugsweise 8 bis 18 C-Atomen und durchschnittlich 4 bis 12 Mol Ethylenoxid (EO) pro Mol Alkohol eingesetzt, in denen der Alkoholrest linear ist. Insbesondere sind Alkoholethoxylate mit 12 bis 18 C-Atomen, zum Beispiel aus Kokos-, Palm-, Talgfett- oder Oleylalkohol, und durchschnittlich 5 bis 8 EO pro Mol Alkohol bevorzugt. Zu den bevorzugten ethoxylierten Alkoholen gehören beispielsweise C₁₂₋₁₄-Alkohole mit 4 EO oder 7 EO, C₉₋₁₁-Alkohol mit 7 EO, C₁₂₋₁₈-Alkohole mit 5 EO oder 7 EO und Mischungen aus diesen. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Geeignet sind ferner auch eine Mischung aus einem (stärker) verzweigten ethoxylierten Fettalkohol und einem unverzweigten ethoxylierten Fettalkohol, wie beispielsweise eine Mischung aus einem C₁₆₋₁₈-Fettalkohol mit 7 EO und 2-Propylheptanol mit 7 EO. Die Menge an nichtionischem Tensid beträgt vorzugsweise bis zu 25 Gew.-%, insbesondere 1 Gew.-% bis 20 Gew.-%, wobei die Angabe von Gew.-% hier und im Folgenden jeweils auf das gesamte Waschmittel bezogen ist, sofern nicht anders angegeben.

Gegebenenfalls zusätzlich vorhandene anionische Tenside umfassen Alkylbenzolsulfonsäuresalze, Olefinsulfonsäuresalze, C₁₂₋₁₈-Alkansulfonsäuresalze, Salze von Schwefelsäuremonoestern mit einem Fettalkohol, Fettsäureseifen, Salze von Schwefelsäuremonoestern mit einem ethoxylierten Fettalkohol oder eine Mischung aus zwei oder mehreren dieser anionischen Tenside.

Als Tenside vom Sulfonat-Typ kommen dabei zum Beispiel C₉₋₁₃-Alkylbenzolsulfonate, Olefinsulfonate, das heißt Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch C₁₂₋₁₈-Alkansulfonate und die Ester von α-Sulfofettsäuren (Estersulfonate), zum Beispiel die α-sulfonierten Methylester der hydrierten Kokos-, Palmkern- oder Talgfettsäuren.

Als Alk(en)ylsulfate werden die Salze der Schwefelsäurehalbester der C₁₂-C₁₈-Fettatkohote, beispielsweise aus Kokosfettalkohol, Talgfettalkohol, Lauryl-, Myristyl-, Cetyl- oder Stearylalkohol oder der C₁₀-C₂₀-Oxo-Alkohote und diejenigen Halbester sekundärer Alkohole dieser Kettenlängen bevorzugt. Aus waschtechnischem Interesse sind die C₁₂-C₁₆-Alkylsulfate und C₁₂-C₁₅-Alkylsulfate sowie C₁₄-C₁₅-Alkylsulfate bevorzugt.

Auch Fettalkoholethersulfate, wie die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁Alkohole mit im Durchschnitt 3,5 Mol Ethylenoxid (EO) oder C₁₂₋₁₈-Fettalkohole mit 1 bis 4 EO, sind geeignet.

Weitere geeignete anionische Tenside sind Fettsäureseifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die zusätzlichen anionischen Tenside einschließlich der Fettsäureseifen können in Form ihrer Natrium-, Kalium- oder Magnesium- oder Ammoniumsalze vorliegen. Vorzugsweise liegen die anionischen Tenside in Form ihrer Natriumsalze oder Ammoniumsalze vor. Zur Neutralisation einsetzbare Amine sind vorzugsweise Cholin, Triethylamin, Monoethanolamin, Diethanolamin, Triethanolamin, Methylethylamin oder eine Mischung daraus, wobei Monoethanolamin bevorzugt ist. In einer besonders bevorzugten Ausführungsformen enthält das Mittel, insbesondere wenn es in flüssiger Form vorliegt, mit Monoethanolamin neutralisierte Alkylbenzolsulfonsäure, insbesondere C₉₋₁₃-Alkylbenzolsulfonsäure, und/oder mit Monoethanolamin neutralisierte Fettsäure.

Der Gehalt an zusätzlichem anionischem Tensid, falls ein solches vorhanden ist, beträgt in dem erfindungsgemäßen Mittel vorzugsweise bis zu 30 Gew.-%, insbesondere 1 Gew.-% bis 25 Gew.-%.

Ein erfindungsgemäßes Mittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Glycindiessigsäure, Methylglycindiessigsäure, Nitrilotriessigsäure, Iminodisuccinate wie Ethylendiamin-N,N'-dibernsteinsäure und Hydroxyiminodisuccinate, Ethylendiamintetraessigsäure sowie Polyasparaginsäure, Polyphosphonsäuren, insbesondere Aminotris(methylenphosphonsäure), Ethylendiamintetrakis(methylen-phosphonsäure), Lysintetra(methylenphosphonsäure) und 1-Hydroxyethan-1,1-diphosphonsäure, polymere Hydroxyverbindungen wie Dextrin sowie polymere (Poly-)carbonsäuren, insbesondere durch Oxidation von Polysacchariden zugängliche Polycarboxylate, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative mittlere Molekülmasse der Homopolymeren ungesättigter Carbonsäuren liegt im Allgemeinen zwischen 5 000 g/mol und 200 000 g/mol, die der Copolymeren zwischen 2 000 g/mol und 200 000 g/mol, vorzugsweise 50 000 g/mol bis 120 000 g/mol, jeweils bezogen auf freie Säure. Ein besonders bevorzugtes Acrylsäure-Maleinsäure-Copolymer weist eine relative mittlere Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/ oder ein Vinylalkohol-Derivat oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäue, insbesondere von (Meth)-acrylsäure ab. Das zweite saure Monomer beziehungsweise dessen Salz kann ein Derivat einer C₄-C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von C₁-C₄-Carbonsäuren, mit Vinylalkohol darstellen. Bevorzugte Polymere enthalten dabei 60 Gew.-% bis 95 Gew.-%, insbesondere 70 Gew.-% bis 90 Gew.-% (Meth)acrylsäure oder (Meth)acrylat, besonders bevorzugt Acrylsäure oder Acrylat, und Maleinsäure oder Maleinat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Polymere, in denen das Gewichtsverhältnis von (Meth)acrylsäure beziehungsweise (Meth)acrylat zu Maleinsäure beziehungsweise Maleinat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem C₁-C₄-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)acrylsäure beziehungsweise (Meth)acrylat, besonders bevorzugt Acrylsäure beziehungsweise Acrylat, 10 Gew.-% bis 30 Gew.-%, vorzugsweise 15 Gew.-% bis 25 Gew.-% Methallylsulfonsäure oder Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di, Oligo- oder Polysaccharid sein, wobei Mono-, Di- oder Oligosaccharide bevorzugt sind. Besonders bevorzugt ist Saccharose. Durch den Einsatz des dritten Monomers werden vermutlich Sollbruchstellen in das Polymer eingebaut, die für die gute biologische Abbaubarkeit des Polymers verantwortlich sind. Diese Terpolymere weisen im Allgemeinen eine relative mittlere Molekülmasse zwischen 1 000 g/mol und 200 000 g/mol, vorzugsweise zwischen 200 g/mol und 50 000 g/mol auf. Weitere bevorzugte Copolymere sind solche, die als Monomere Acrolein und Acrylsäure/ Acrylsäuresalze beziehungsweise Vinylacetat aufweisen. Die organischen Buildersubstanzen können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen in der oberen Hälfte der genannten Bereiche werden vorzugsweise in pastenförmigen oder flüssigen, insbesondere wasserhaltigen, Mitteln eingesetzt.

Als wasserlösliche anorganische Buildermaterialien kommen insbesondere Polyphosphate, vorzugsweise Natriumtriphosphat, in Betracht. Als wasserunlösliche anorganische Buildermaterialien werden insbesondere kristalline oder amorphe, wasserdispergierbare Alkalialumosilikate, in Mengen nicht über 25 Gew.-%, vorzugsweise von 3 Gew.-% bis 20 Gew.-% und insbesondere in Mengen von 5 Gew.-% bis 15 Gew.-% eingesetzt. Unter diesen sind die kristallinen Natriumalumosilikate in Waschmittelqualität, insbesondere Zeolith A, Zeolith P sowie Zeolith MAP und gegebenenfalls Zeolith X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen liegt in der Regel im Bereich von 100 bis 200 mg CaO pro Gramm.

Zusätzlich oder alternativ zum genannten wasserunlöslichen Alumosilikat und Alkalicarbonat können weitere wasserlösliche anorganische Buildermaterialien enthalten sein. Zu diesen gehören neben den Polyphosphaten wie Natriumtriphosphat insbesondere die wasserlöslichen kristallinen und/oder amorphen Alkalisilikat-Builder. Derartige wasserlösliche anorganische Buildermaterialien sind in den Mitteln vorzugsweise in Mengen von 1 Gew.-% bis 20 Gew.-%, insbesondere von 5 Gew.-% bis 15 Gew.-% enthalten. Die als Buildermaterialien brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ ·y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl ß- als auch δ-Natriumdisilikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in den Mitteln eingesetzt werden. In einer weiteren bevorzugten Ausführungsform wird ein kristallines Natriumschichtsilikat mit einem Modul von 2 bis 3 eingesetzt, wie es aus Sand und Soda hergestellt werden kann. Natriumsilikate mit einem Modul im Bereich von 1,9 bis 3,5 werden in einer weiteren Ausführungsform eingesetzt. In einer bevorzugten Ausgestaltung solcher Mittel setzt man ein granulares Compound aus Alkalisilikat und Alkalicarbonat ein, wie es zum Beispiel unter dem Namen Nabion^{®} 15 im Handel erhältlich ist.

Als geeignete peroxidische Bleichmittel kommen insbesondere organische Persäuren oder persaure Salze organischer Säuren, wie Phthalimidopercapronsäure, Perbenzoesäure, Monoperoxyphthalsäure, und Diperdodecandisäure sowie deren Salze wie Magnesiummonoperoxyphthalat, Diacylperoxide, Wasserstoffperoxid und unter den Einsatzbedingungen Wasserstoffperoxid abgebende anorganische Salze, wie Alkaliperborat, Alkalipercarbonat und/oder Alkalipersilikat, und Wasserstoffperoxid-Einschlussverbindungen, wie H₂O₂-Harnstoffaddukte, sowie Mischungen aus diesen in Betracht. Wasserstoffperoxid kann dabei auch mit Hilfe eines enzymatischen Systems, das heißt einer Oxidase und ihres Substrats, erzeugt werden. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder Wasserstoffperoxid eingesetzt. Ein in im Rahmen der Erfindung einsetzbares Waschmittel enthält peroxidisches Bleichmittel in Mengen von vorzugsweise bis zu 60 Gew.-%, insbesondere von 5 Gew.-% bis 50 Gew.-% und besonders bevorzugt von 15 Gew.-% bis 30 Gew.-% oder alternativ von 2,5 Gew.-% bis 20 Gew.-%, wobei in flüssigen Mitteln Wasserstoffperoxid und in festen Mitteln Natriumpercarbonat das besonders bevorzugte peroxidische Bleichmittel ist. Vorzugsweise weisen peroxidische Bleichmittel-Partikel eine Teilchengröße im Bereich von 10 µm bis 5000 µm, insbesondere von 50 µm bis 1000 µm und/oder eine Dichte von 0,85 g/cm³ bis 4,9 g/cm³, insbesondere von 0,91 g/cm³ bis 2,7 g/cm³ auf.

Als bleichaktivierende, unter Perhydrolysebedingungen Peroxocarbonsäure-liefernde Verbindung können insbesondere Verbindungen, die unter Perhydrolysebedingungen gegebenenfalls substituierte Perbenzoesäure und/oder aliphatische Peroxocarbonsäuren mit 1 bis 12 C-Atomen, insbesondere 2 bis 4 C-Atomen ergeben, allein oder in Mischungen, eingesetzt werden. Geeignet sind Bleichaktivatoren, die O- und/oder N-Acylgruppen insbesondere der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Glykolurile, insbesondere Tetraacetylglykoluril (TAGU), acylierte Triazinderivate, insbesondere 1,5-Diacetyl-2,4-dioxohexahydro-1,3,5-triazin (DADHT), N-Acylimide, insbesondere N-Nonanoylsuccinimid (NOSI), acylierte Phenolsulfonate oder -carboxylate beziehungsweise die Sulfon- oder Carbonsäuren von diesen, insbesondere Nonanoyl- oder Isononanoyl- oder Lauroyloxybenzolsulfonat (NOBS beziehungsweise iso-NOBS beziehungsweise LOBS) oder Decanoyloxybenzoat (DOBA), deren formale Kohlensäureesterderivate wie 4-(2-Decanoyloxyethoxycarbonyloxy)-benzolsulfonat (DECOBS), acylierte mehrwertige Alkohole, insbesondere Triacetin, Ethylenglykoldiacetat und 2,5-Di-acetoxy-2,5-dihydrofuran sowie acetyliertes Sorbitol und Mannitol und deren Mischungen (SORMAN), acylierte Zuckerderivate, insbesondere Pentaacetylglukose (PAG), Pentaacetyl-fruktose, Tetraacetylxylose und Octaacetyllactose, acetyliertes, gegebenenfalls N-alkyliertes Glucamin und Gluconolacton, und/oder N-acylierte Lactame, beispielsweise N-Benzoylcaprolactam.

Zusätzlich zu den Verbindungen, die unter Perhydrolysebedingungen Peroxocarbonsäuren bilden, oder an deren Stelle können weitere bleichaktivierende Verbindungen, wie beispielsweise Nitrile, aus denen sich unter Perhydrolysebedingungen Perimidsäuren bilden, vorhanden sein. Dazu gehören insbesondere Aminoacetonitrilderivate mit quaterniertem Stickstoffatom gemäß der Formel 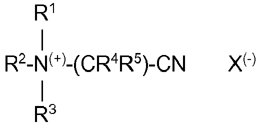 in der R¹ für -H, -CH₃, einen C₂₋₂₄-Alkyl- oder -Alkenylrest, einen substituierten C₁₋₂₄-Alkyl- oder C₂₋₂₄-Alkenylrest mit mindestens einem Substituenten aus der Gruppe -Cl, -Br, -OH, -NH₂, -CN und - N⁽⁺⁾-CH₂-CN, einen Alkyl- oder Alkenylarylrest mit einer C₁₋₂₄-Alkylgruppe, oder für einen substituierten Alkyl- oder Alkenylarylrest mit mindestens einer, vorzugsweise zwei, gegebenenfalls substituierten C₁₋₂₄-Alkylgruppe(n) und gegebenenfalls weiteren Substituenten am aromatischen Ring steht, R² und R³ unabhängig voneinander ausgewählt sind aus -CH₂-CN, -CH₃, -CH₂-CH₃, CH₂-CH2-CH₃, - CH(CH₃)-CH₃,-CH₂-OH, -CH₂-CH₂-OH, -CH(OH)-CH₃, -CH₂-CH₂-CH₂-OH, -CH₂-CH(OH)-CH₃, - CH(OH)-CH₂-CH₃, -(CH₂CH₂-O)ₙH mit n = 1, 2, 3, 4, 5 oder 6, R⁴ und R⁵ unabhängig voneinander eine voranstehend für R¹, R² oder R³ angegebene Bedeutung haben, wobei mindestens 2 der genannten Reste, insbesondere R² und R³, auch unter Einschluss des Stickstoffatoms und gegebenenfalls weiterer Heteroatome ringschließend miteinander verknüpft sein können und dann vorzugsweise einen Morpholino-Ring ausbilden, und X ein ladungsausgleichendes Anion, vorzugsweise ausgewählt aus Benzolsulfonat, Toluolsulfonat, Cumolsulfonat, den C₉₋₁₅-Alkylbenzolsulfonaten, den C₁₋₂₀-Alkylsulfaten, den C₈₋₂₂-Carbonsäure-methylestersulfonaten, Sulfat, Hydrogensulfat und deren Gemischen, ist, können eingesetzt werden. Unter Perhydrolysebedingungen Peroxocarbonsäuren oder Perimidsäuren bildende Bleichaktivatoren sind vorzugsweise in Mengen bis zu 25 Gew.%, insbesondere 0,1 Gew.-% bis 10 Gew.-% in erfindungsgemäßen Mitteln vorhanden. Vorzugsweise weisen Bleichaktivator-Partikel eine Teilchengröße im Bereich von 10 µm bis 5000 µm, insbesondere von 50 µm bis 1000 µm und/oder eine Dichte von 0,85 g/cm³ bis 4,9 g/cm³, insbesondere von 0,91 g/cm³ bis 2,7 g/cm³ auf.

Die Anwesenheit von bleichkatalysierenden Übergangsmetallkomplexen, zusätzlich zu oder an Stelle von den genannten Bleichaktivatoren, ist möglich. Diese werden vorzugsweise unter den Cobalt-, Eisen-, Kupfer-, Titan-, Vanadium-, Mangan- und Rutheniumkomplexen ausgewählt. Als Liganden in derartigen Übergangsmetallkomplexen kommen sowohl anorganische als auch organische Verbindungen in Frage, zu denen neben Carboxylaten insbesondere Verbindungen mit primären, sekundären und/oder tertiären Amin- und/oder Alkohol-Funktionen, wie Pyridin, Pyridazin, Pyrimidin, Pyrazin, Imidazol, Pyrazol, Triazol, 2,2'-Bispyridylamin, Tris-(2-pyridylmethyl)amin, 1,4,7-Triazacyclononan, 1,4,7-Trimethyl-1,4,7-triazacyclononan, 1,5,9-Trimethyl-1,5,9-triazacyclodode-can, (Bis-((1-methylimidazol-2-yl)-methyl))-(2-pyridylmethyl)-amin, N,N'-(Bis-(1-methylimidazol-2-yl)-methyl)-ethylendiamin, N-Bis-(2-benzimidazolylmethyl)-aminoethanol, 2,6-Bis-(bis-(2-benzimida-zolylmethyl)aminomethyl)-4-methylphenol, N,N,N',N'-Tetrakis-(2-benzimidazolylmethyl)-2-hydroxy-1,3-diaminopropan, 2,6-Bis-(bis-(2-pyridylmethyl)aminomethyl)-4-methylphenol, 1,3-Bis-(bis-(2-benzimidazolyl-methyl)aminomethyl)-benzol, Sorbitol, Mannitol, Erythritol, Adonitol, Inositol, Lactose, und gegebenenfalls substituierte Salene, Porphine und Porphyrine gehören. Zu den anorganischen Neutralliganden gehören insbesondere Ammoniak und Wasser. Falls nicht sämtliche Koordinationsstellen des Übergangsmetallzentralatoms durch Neutralliganden besetzt sind, enthält der Komplex weitere, vorzugsweise anionische und unter diesen insbesondere ein- oder zweizähnige Liganden. Zu diesen gehören insbesondere die Halogenide wie Fluorid, Chlorid, Bromid und lodid, und die (NO₂)⁻-Gruppe, das heißt ein Nitro-Ligand oder ein Nitrito-Ligand. Die (NO₂)⁻-Gruppe kann an ein Übergangsmetall auch chelatbildend gebunden sein oder sie kann zwei Übergangsmetallatome asymmetrisch oder µ1-O-verbrücken. Außer den genannten Liganden können die Übergangsmetallkomplexe noch weitere, in der Regel einfacher aufgebaute Liganden, insbesondere ein- oder mehrwertige Anionliganden, tragen. In Frage kommen beispielsweise Nitrat, Acetat, Trifluoracetat, Formiat, Carbonat, Citrat, Oxalat, Perchlorat sowie komplexe Anionen wie Hexafluorophosphat. Die Anionliganden sollen für den Ladungsausgleich zwischen Übergangsmetall-Zentralatom und dem Ligandensystem sorgen. Auch die Anwesenheit von Oxo-Liganden, Peroxo-Liganden und Imino-Liganden ist möglich. Insbesondere derartige Liganden können auch verbrückend wirken, so dass mehrkernige Komplexe entstehen. Im Falle verbrückter, zweikerniger Komplexe müssen nicht beide Metallatome im Komplex gleich sein. Auch der Einsatz zweikerniger Komplexe, in denen die beiden Übergangsmetallzentralatome unterschiedliche Oxidationszahlen aufweisen, ist möglich. Falls Anionliganden fehlen oder die Anwesenheit von Anionliganden nicht zum Ladungsausgleich im Komplex führt, sind in den gemäß der Erfindung zu verwendenden Übergangsmetallkomplex-Verbindungen anionische Gegenionen anwesend, die den kationischen Übergangsmetall-Komplex neutralisieren. Zu diesen anionischen Gegenionen gehören insbesondere Nitrat, Hydroxid, Hexafluorophosphat, Sulfat, Chlorat, Perchlorat, die Halogenide wie Chlorid oder die Anionen von Carbonsäuren wie Formiat, Acetat, Oxalat, Benzoat oder Citrat. Beispiele für einsetzbare Übergangsmetallkomplex-Verbindungen sind [N,N'-Bis[(2-hydroxy-5-vinylphenyl)-methylen]-1,2-diamino-cyclohexan]-mangan-(III)-chlorid, [N,N'-Bis[(2-hydroxy-5-nitrophenyl)-methylen]-1,2-diamino-cyclohexan]-mangan-(III)-acetat, [N,N'-Bis[(2-hydroxyphenyl)-methylen]-1,2-phenylendiamin]-mangan-(III)-acetat, [N,N'-Bis[(2-hydroxyphenyl)-methylen]-1,2-diaminocyclohexan]-mangan-(III)-chlorid, [N,N'-Bis[(2-hydroxyphenyl)-methylen]-1,2-diaminoethan]-mangan-(III)-chlorid, [N,N'-Bis[(2-hydroxy-5-sulfonatophe-nyl)-methylen]-1,2-diaminoethan]-mangan-(III)-chlorid, Mangan-oxalatokomplexe, Nitropentammin-cobalt(III)-chlorid, Nitritopentammin-cobalt(III)-chlorid, Hex-ammincobalt(III)-chlorid, Chloropentam-min-cobalt(III)-chlorid sowie der Peroxo-Komplex [(NH₃)₅Co-O-O-Co(NH₃)₅]Cl₄.

Als in den Mitteln verwendbare Enzyme kommen solche aus der Klasse der Proteasen, Amylasen, Lipasen, Cutinasen, Pullulanasen, Hemicellulasen, Cellulasen, Oxidasen, Laccasen und Peroxidasen sowie deren Gemische in Frage. Besonders geeignet sind aus Pilzen oder Bakterien, wie Bacillus subtilis, Bacillus licheniformis, Bacillus lentus, Streptomyces griseus, Humicola lanuginosa, Humicola insolens, Pseudomonas pseudoalcaligenes, Pseudomonas cepacia oder Coprinus cinereus gewonnene enzymatische Wirkstoffe. Die Enzyme können an Trägerstoffen adsorbiert und/oder in Hüllsubstanzen eingebettet sein, um sie gegen vorzeitige Inaktivierung zu schützen. Sie sind in den erfindungsgemäßen Wasch- oder Reinigungsmitteln vorzugsweise in Mengen bis zu 5 Gew.-%, insbesondere von 0,002 Gew.-% bis 4 Gew.-%, enthalten. Falls das erfindungsgemäße Mittel Protease enthält, weist es vorzugsweise eine proteolytische Aktivität im Bereich von etwa 100 PE/g bis etwa 10 000 PE/g, insbesondere 300 PE/g bis 8000 PE/g auf. Falls mehrere Enzyme in dem erfindungsgemäßen Mittel eingesetzt werden sollen, kann dies durch Einarbeitung der zwei oder mehreren separaten beziehungsweise in bekannter Weise separat konfektionierten Enzyme oder durch zwei oder mehrere gemeinsam in einem Granulat konfektionierte Enzyme durchgeführt werden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den erfindungsgemäßen Mitteln in Mengen von vorzugsweise nicht über 20 Gew.%, insbesondere von 1,2 Gew.-% bis 17 Gew.-%, enthalten.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.%, bezogen auf die Mittel, eingesetzt.

Die Mittel können gewünschtenfalls einen üblichen Farbübertragungsinhibitor, diesen dann vorzugsweise in Mengen bis zu 2 Gew.-%, insbesondere 0,1 Gew.-% bis 1 Gew.-%, enthalten, der in einer bevorzugten Ausgestaltung ausgewählt wird aus den Polymeren aus Vinylpyrrolidon, Vinylimidazol, Vinylpyridin-N-Oxid oder den Copolymeren aus diesen. Brauchbar sind sowohl Polyvinylpyrrolidone mit Molgewichten von 15 000 g/mol bis 50 000 g/mol wie auch Polyvinylpyrrolidone mit höheren Molgewichten von beispielsweise bis zu über 1 000 000 g/mol, insbesondere von 1 500 000 g/mol bis 4 000 000 g/mol, N-Vinylimidazol/N-Vinylpyrrolidon-Copolymere, Polyvinyloxazolidone, Copolymere auf Basis von Vinylmonomeren und Carbonsäureamiden, pyrrolidongruppenhaltige Polyester und Polyamide, gepfropfte Polyamidoamine und Polyethylenimine, Polyamin-N-Oxid-Polymere und Polyvinylalkohole. Eingesetzt werden können aber auch enzymatische Systeme, umfassend eine Peroxidase und Wasserstoffperoxid beziehungsweise eine in Wasser Wasserstoffperoxid-liefernde Substanz. Der Zusatz einer Mediatorverbindung für die Peroxidase, zum Beispiel eines Acetosyringons, eines Phenolderivats oder eines Phenotiazins oder Phenoxazins, ist in diesem Fall bevorzugt, wobei auch zusätzlich obengenannte polymere Farbübertragungsinhibitorwirkstoffe eingesetzt werden können. Polyvinylpyrrolidon weist vorzugsweise eine durchschnittliche Molmasse im Bereich von 10 000 g/mol bis 60 000 g/mol, insbesondere im Bereich von 25 000 g/mol bis 50 000 g/mol auf. Unter den Copolymeren sind solche aus Vinylpyrrolidon und Vinylimidazol im Molverhältnis 5:1 bis 1:1 mit einer durchschnittlichen Molmasse im Bereich von 5 000 g/mol bis 50 000 g/mol, insbesondere 10 000 g/mol bis 20 000 g/mol bevorzugt. In bevorzugten Ausführungsformen der Erfindung sind die Waschmittel allerdings frei von derartigen zusätzlichen Farbübertragungsinhibitoren.

Waschmittel können als optische Aufheller beispielsweise Derivate der Diaminostilbendisulfonsäure beziehungsweise deren Alkalimetallsalze enthalten, obgleich sie für den Einsatz als Colorwaschmittel vorzugsweise frei von optischen Aufhellern sind. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Insbesondere beim Einsatz in maschinellen Verfahren kann es von Vorteil sein, den Mitteln übliche Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bisfettsäurealkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinen und Bistearylethylendiamid bevorzugt.

In einer bevorzugten Ausführungsform ist das erfindungsgemäße Mittel teilchenförmig und enthält neben dem Tensid der allgemeinen Formel (I) Builder, insbesondere in einer Menge im Bereich von 1 Gew.-% bis 60 Gew.-%.

In einer weiteren bevorzugten Ausführungsform ist ein erfindungsgemäßes Mittel flüssig und enthält 1 Gew.-% bis 90 Gew.-%, insbesondere 10 Gew.-% bis 85 Gew.-%, bevorzugt 25 Gew.-% bis 75 Gew.-%, und besonders bevorzugt 35 Gew.-% bis 65 Gew.-% Wasser, wassermischbares Lösungsmittel oder eines Gemisches aus Wasser und wassermischbarem Lösungsmittel. Zu wassermischbaren Lösungsmitteln gehören beispielsweise einwertige Alkohole mit 1 bis 4 C-Atomen, insbesondere Methanol, Ethanol, Isopropanol und tert.-Butanol, Diole und Triole mit 2 bis 4 C-Atomen, insbesondere Ethylenglykol, Propylenglykol und Glycerin, sowie deren Gemische und die aus den genannten Verbindungsklassen ableitbaren Ether. Derartige wassermischbare Lösungsmittel sind in den erfindungsgemäßen Mitteln vorzugsweise in Mengen nicht über 30 Gew.-%, insbesondere von 2 Gew.-% bis 20 Gew.-%, vorhanden.

In einer weiteren bevorzugten Ausführungsform liegt das erfindungsgemäße Mittel einzeldosierfertig portioniert in einer aus wasserlöslichem Material gebildeten Kammer vor; dann enthält das Mittel vorzugsweise weniger als 15 Gew.-%, insbesondere im Bereich von 1 Gew.-% bis 12 Gew.-% Wasser. Eine Portion ist eine eigenständige Dosiereinheit mit mindestens einer Kammer, in der zu dosierendes Gut enthalten ist. Eine Kammer ist ein durch Wandungen (zum Beispiel durch eine Folie) abgegrenzter Raum, welcher auch ohne das zu dosierende Gut (gegebenenfalls unter Veränderung seiner Form) existieren kann. Bei einer Oberflächenbeschichtung oder einer Schicht einer Oberflächenbeschichtung handelt es sich somit nicht um eine Wandung gemäß der vorliegenden Erfindung.

Dabei sind die Wandungen der Kammer aus einem wasserlöslichen Material. Die Wasserlöslichkeit des Materials kann mit Hilfe eines in einem quadratischen Rahmen (Kantenlänge auf der Innenseite: 20 mm) fixierten quadratischen Films des besagten Materials (Film: 22 x 22 mm mit einer Dicke von 76 µm) nach dem folgenden Messprotokoll bestimmt werden. Besagter gerahmter Film wird in 800 ml auf 20 °C temperiertes, destilliertes Wasser in einem 1 Liter Becherglas mit kreisförmiger Bodenfläche (Fa. Schott, Mainz, Becherglas 1000 ml, niedrige Form) eingetaucht, so dass die Fläche des eingespannten Films im rechten Winkel zur Bodenfläche des Becherglases angeordnet ist, die Oberkante des Rahmens 1 cm unter der Wasseroberfläche ist und die Unterkante des Rahmens parallel zur Bodenfläche des Becherglases derart ausgerichtet ist, dass die Unterkante des Rahmens entlang des Radius der Bodenfläche des Becherglases verläuft und die Mitte der Unterkante des Rahmens über der Mitte des Radius des Becherglasbodens angeordnet ist. Das Material löst sich unter Rühren (Rührgeschwindigkeit Magnetrührer 300 rpm, Rührstab: 5 cm lang) innerhalb von 600 Sekunden derart auf, dass mit dem bloßen Auge keine einzelnen festförmigen Partikel mehr sichtbar sind.

Die Wandungen der Kammern und damit die wasserlöslichen Umhüllungen der erfindungsgemäßen Waschmittel werden vorzugsweise durch ein wasserlösliches Folienmaterial gebildet. Solche wasserlöslichen Verpackungen können entweder durch Verfahren des vertikalen Formfüllversiegelns oder durch Warmformverfahren hergestellt werden.

Das Warmformverfahren schließt im Allgemeinen das Formen einer ersten Lage aus einem wasserlöslichen Folienmaterial zum Bilden von Ausbuchtungen zum Aufnehmen einer Zusammensetzung darin, Einfüllen der Zusammensetzung in die Ausbuchtungen, Bedecken der mit der Zusammensetzung gefüllten Ausbuchtungen mit einer zweiten Lage aus einem wasserlöslichen Folienmaterial und Versiegeln der ersten und zweiten Lagen miteinander zumindest um die Ausbuchtungen herum ein.

Das wasserlösliche Folienmaterial wird vorzugsweise ausgewählt aus Polymeren oder Polymergemischen. Die Umhüllung kann aus einer oder aus zwei oder mehr Lagen von wasserlöslichem Folienmaterial gebildet werden. Die wasserlöslichen Folienmaterialien der ersten Lage und der weiteren Lagen, falls vorhanden, können gleich oder unterschiedlich sein.

Es ist bevorzugt, dass die wasserlösliche Umhüllung Polyvinylalkohol oder ein Polyvinylalkoholcopolymer enthält; besonders bevorzugt besteht sie aus Polyvinylalkohol oder Polyvinylalkoholcopolymer.

Wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 10.000 bis 1.000.000 gmol⁻¹, vorzugsweise von 20.000 bis 500.000 gmol⁻¹, besonders bevorzugt von 30.000 bis 100.000 gmol⁻¹ und insbesondere von 40.000 bis 80.000 gmol⁻¹ liegt.

Die Herstellung von Polyvinylalkohol geschieht üblicherweise durch Hydrolyse von Polyvinylacetat, da der direkte Syntheseweg nicht möglich ist. Ähnliches gilt für Polyvinylalkoholcopolymere, die aus entsprechend aus Polyvinylacetatcopolymeren hergestellt werden. Bevorzugt ist, wenn wenigstens eine Lage der wasserlöslichen Umhüllung einen Polyvinylalkohol umfasst, dessen Hydrolysegrad 70 bis 100 Mol-%, vorzugsweise 80 bis 90 Mol-%, besonders bevorzugt 81 bis 89 Mol-% und insbesondere 82 bis 88 Mol-% ausmacht.

Einem zur Herstellung der wasserlöslichen Umhüllung geeigneten Folienmaterial können zusätzlich Polymere, ausgewählt aus der Gruppe umfassend Acrylsäure-haltige Polymere, Polyacrylamide, Oxazolin-Polymere, Polystyrolsulfonate, Polyurethane, Polyester, Polyether, Polymilchsäure, und/oder Mischungen der vorstehenden Polymere, zugesetzt sein. Auch die Copolymerisation von solchen Polymeren zugrundeliegenden Monomeren, einzeln oder in Mischungen aus zweien oder mehreren, mit Vinylacetat ist möglich.

Bevorzugte Polyvinylalkoholcopolymere umfassen neben Vinylalkohol eine ethylenisch ungesättigte Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus; unter den Estern sind C₁₋₄-Alkylester oder -Hydroxyalkylester bevorzugt. Ebenso bevorzugte Polyvinylalkoholcopolymere umfassen neben Vinylalkohol als weitere Monomere ethylenisch ungesättigte Dicarbonsäuren. Geeignete Dicarbonsäure sind beispielsweise Itaconsäure, Maleinsäure, Fumarsäure und Mischungen daraus, wobei Itaconsäure besonders bevorzugt ist.

Geeignete wasserlösliche Folien zum Einsatz in den Umhüllungen der wasserlöslichen Verpackungen gemäß der Erfindung sind Folien, die von der Firma MonoSol LLC beispielsweise unter der Bezeichnung M8630, C8400 oder M8900 vertrieben werden. Andere geeignete Folien umfassen Folien mit der Bezeichnung Solublon^{®} PT, Solublon^{®} GA, Solublon^{®} KC oder Solublon^{®} KL von der Aicello Chemical Europe GmbH oder die Folien VF-HP von Kuraray.

Die Wasch- oder Reinigungsmittelportion, umfassend das Wasch- oder Reinigungsmittel und die wasserlösliche Umhüllung, kann eine oder mehr Kammern aufweisen. Die wasserlöslichen Umhüllungen mit einer Kammer können eine im Wesentlichen formstabile kugel-, rotationsellipsoid-, würfel, quader- oder kissenförmige Ausgestaltung mit einer kreisförmigen, elliptischen, quadratischen oder rechteckigen Grundform aufweisen. Das Mittel kann in einer oder mehreren Kammern, falls vorhanden, der wasserlöslichen Umhüllung enthalten sein.

In einer bevorzugten Ausführungsform weist die wasserlösliche Umhüllung zwei Kammern auf. In dieser Ausführungsform können beide Kammern jeweils eine feste Teilzusammensetzung oder jeweils eine flüssige Teilzusammensetzung enthalten, oder die erste Kammer enthält eine flüssige und die zweite Kammer eine feste Teilzusammensetzung.

Die Anteile der Mittel, die in den unterschiedlichen Kammern einer wasserlöslichen Umhüllung mit zwei oder mehr Kammern enthalten sind, können dieselbe Zusammensetzung aufweisen. Vorzugsweise weisen die Mittel in einer wasserlöslichen Umhüllung mit mindestens zwei Kammern jedoch Teilzusammensetzungen auf, die sich mindestens in einem Inhaltsstoff und/oder in dem Gehalt mindestens eines Inhaltsstoffes unterscheiden. Vorzugsweise weist eine Teilzusammensetzung solcher erfindungsgemäßer Mittel Enzym und/oder Bleichaktivator auf und eine getrennt davon vorliegende weitere Teilzusammensetzung weist peroxidisches Bleichmittel auf, wobei dann die erstgenannte Teilzusammensetzung insbesondere kein peroxidisches Bleichmittel und die zweitgenannte Teilzusammensetzung insbesondere kein Enzym und keinen Bleichaktivator aufweist.

Durch die portionsweise Verpackung in eine wasserlösliche Umhüllung wird der Anwender in die Lage versetzt, für eine Anwendung eine oder gewünschtenfalls mehrere, vorzugsweise eine, der Portionen in die Wasch- oder Geschirrspülmaschine, insbesondere in die Einspülkammer einer Waschmaschine, oder in ein Behältnis zur Durchführung eines manuellen Wasch- oder Reinigungsverfahrens zu geben. Derartige Portionspackungen erfüllen den Wunsch des Verbrauchers nach vereinfachter Dosierung. Nach Zugabe von Wasser löst sich das Umhüllungsmaterial auf, so dass die Inhaltsstoffe freigesetzt werden und in der Flotte ihre Wirkung entfalten können. Vorzugsweise wiegt eine wasserlöslich umhüllte Portion 10 g bis 35 g, insbesondere 12 g bis 28 g und besonders bevorzugt 12 g bis 15 g, wobei auf den in der Gewichtsangabe enthaltenen Anteil der wasserlöslichen Umhüllung 0,3 g bis 2,5 g, insbesondere 0,7 g bis 1,2 g entfallen.

Die Herstellung fester erfindungsgemäßer Mittel bietet keine Schwierigkeiten und kann auf bekannte Weise, zum Beispiel durch Sprühtrocknen oder Granulation, erfolgen, wobei Enzyme und eventuelle weitere thermisch empfindliche Inhaltsstoffe wie zum Beispiel Bleichmittel gegebenenfalls später separat zugesetzt werden. Zur Herstellung von Mitteln mit erhöhtem Schüttgewicht, insbesondere im Bereich von 650 g/l bis 950 g/l, ist ein einen Extrusionsschritt aufweisendes Verfahren bevorzugt.

Flüssige beziehungsweise pastöse erfindungsgemäße Mittel in Form von Wasser übliche Lösungsmittel enthaltenden Lösungen werden in der Regel durch einfaches Mischen der Inhaltsstoffe, die in Substanz oder als Lösung in einen automatischen Mischer gegeben werden können, hergestellt.

### Beispiele

### Beispiel 1: Synthese von Natrium-1-(Tetrahydrofuran-2-yl)pentadecyl-sulfat (P1)

### A: Herstellung von 1-(Furan-2-yl)pentadecan-1-ol

Zu 6,2 g getrockneter Magnesiumspäne (253 mmol, 1,5 eq) in 169 ml trockenem Diethylether wurden 46,8 g 1-Bromtetradecan (169 mmol, 1 eq) langsam zugetropft. Die Mischung wurde für 3 Stunden unter Rückfluss erhitzt und gerührt. Anschließend wurde das so hergestellte Grignard-Reagenz unter Eiskühlung langsam zu einer Lösung von 12,5 g Furfural (130 mmol, 1 eq) in 50 ml trockenem Diethylether getropft. Nach Erwärmen auf Raumtemperatur und 3 Stunden Rühren unter Rückfluss wurde gesättigte wässrige Ammoniumchloridlösung zugegeben. Zur Isolation des Produktes wurde dieses mit Wasser und Diethylether mehrfach extrahiert. Nach Trocknen im Hochvakuum wurden 37,9 g 1-(Furan-2-yl)pentadecan-1-ol als leicht gelber Feststoff erhalten. Ausbeute: 99 %.

¹H-NMR (400 MHz, CDCl₃): δ (ppm) = 0.88 (t, 3H, H₁₉), 1.27 (m, 22H, H₈₋₁₈), 1.41 (m, 2H, H₇), 1.88 (m, 2H, H₆), 4.65 (t, 1H, H₅), 6.28 (m, 2H, H₂₋₃), 7.36 (m, 1H, H₁).

¹³C-NMR (100 MHz, CDCl₃): δ (ppm) = 14.25 (s, 1C, C₁₉), 22.84 (s, 1C, C₁₈), 25.68 (s, 1C, C₇), 29.85 (m, 9C, C₈₋₁₆), 32.07 (s, 1C, C₁₇), 35.70 (s, 1C, C₆), 67.94 (s, 1C, C₅), 105.83 (s, 1C, C₃), 110.19 (s, 1C, C₂), 141.91 (s, 1C, C₁), 157.09 (s, 1C, C₄).

### B: Herstellung von 1-(Tetrahydrofuran-2-yl)pentadecan-1-ol

Zu einer Lösung von 20 g 1-(Furan-2-yl)pentadecan-1-ol (68 mmol) in 100 ml Ethanol wurden 2 g Ni/SiO₂ gegeben Die Mischung wurde in einen Autoklav überführt und bei einem Wasserstoffdruck von 150 bar und einer Temperatur von 100°C für 3 Stunden gerührt. Anschließend wurde der Katalysator durch Zentrifugieren abgetrennt und 1-(Tetrahydrofuran-2-yl)pentadecan-1-ol durch Entfernen des Lösungsmittels als farblose Flüssigkeit isoliert. Ausbeute: 100%.

¹³C-NMR (100 MHz, CDCl₃): δ (ppm) = 14.21 (s, 1C, C₁₉), 22.80 (s, 1C, C₁₈), 24.58 (s, 1C, C₇), 26.11 (s, 1C, C₂), 26.38 (s, 1C, C₃), 28.07 (s, 1C, C₃), 29.73 (m, 8C, C₉₋₁₆), 32.04 (s, 1C, C₁₇), 33.84 (s, 1C, C₆), 68.38 (d, 1C, C₁), 73.01 (d, 1C, C₃), 82.49 (d, 1C, C₄).

### C: Herstellung von Natrium-1-(Tetrahydrofuran-2-yl)pentadecyl-sulfat

Zu einer Lösung von 14,40 g 1-(Furan-2-yl)pentadecan-1-ol (48 mmol) in 400 ml Acetonitril wurden 11,52 g SO₃Py (72 mmol, 1.5 eq) zugegeben und die Mischung wurde bei 70 °C für 6 Stunden gerührt. Anschließend wurden 0,7 ml Wasser zugegeben und die Mischung wurde für weitere 30 Minuten gerührt. Das Lösungsmittel wurde entfernt und der Rückstand wurde in Ethanol gelöst. 10 g feingemörsertes Na₂CO₃ (96 mmol, 2 eq) wurde hinzugegeben und die Mischung wurde bei 50°C für 2 Stunden gerührt. Der Feststoff wurde über Silicagel abfiltriert und das Filtrat eingeengt. Nach Zugabe von Petrolether fiel Natrium-1-(Tetrahydrofuran-2-yl)pentadecyl-sulfat (P1) als weißer Feststoff aus, der mittels Zentrifuge abgetrennt und im Hochvakuum getrocknet wurde. Ausbeute: 100%.

¹³C-NMR (100 MHz, MeOD): δ (ppm) = 14.45 (s, 1C, C₁₉), 23.73 (s, 1C, C₁₈), 25.81 (s, 1C, C₇), 26.51 (s, 1C, C₂), 26.89 (s, 1C, C₃), 28.87 (s, 1C, C₃), 28.45 (s, 1C, C₉), 30.69 (m, 7C, C₁₀₋₁₆), 32.13 (s, 1C, C₆), 33.08 (s, 1C, C₁₇), 69.49 (d, 1C, C₁), 80.65 (d, 1C, C₄), 81.65 (d, 1C, C₅).

Die kritische Mizellkonzentration (CMC) von Tensid P1 wurde durch Messung der Oberflächenspannung seiner wässrigen Lösung als Funktion der Konzentration bei 25°C und einem pH von 8,5 zu 0,01 g/l bestimmt. Die Grenzflächenspannung einer wässrigen Lösung von P1 (Konzentration 1 g/l) gegenüber Isopropylmyristat bei pH 8,5 und 25°C wurde mittels der Spinning Drop Methode gemessen. Nach 20 Minuten ergab sich ein Wert von 3 mN/m.

### Beispiel 2: Synthese von Natrium-1-(Tetrahydrofuran-2-yl) tridecyl-sulfat (P2)

Beispiel 1 wurde wiederholt, wobei in Schritt A statt 1-Bromtetradecan 1-Bromdodecan eingesetzt wurde.

Die kritische Mizellkonzentration (CMC) von Tensid P2 wurde durch Messung der Oberflächenspannung seiner wässrigen Lösung als Funktion der Konzentration bei 25°C und einem pH von 8,5 zu 0,24 g/l bestimmt. Die Grenzflächenspannung einer wässrigen Lösung von P1 (Konzentration 1 g/l) gegenüber Isopropylmyristat bei pH 8,5 und 25°C wurde mittels der Spinning Drop Methode gemessen. Nach 20 Minuten ergab sich ein Wert von 4 mN/m.

## Patentansprüche

1. Anionisches Tensid der allgemeinen Formel (I), 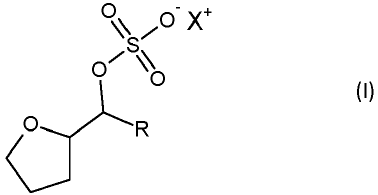 in der R für eine lineare oder verzweigte Alkyl-, Alkenyl-, Alkylaryl- oder Alkenylarylgruppe mit 5 bis 25 C-Atomen und X⁺ für ein ladungsausgleichendes Kation steht.

2. Verfahren zur Herstellung eines anionischen Tensids der allgemeinen Formel (I), 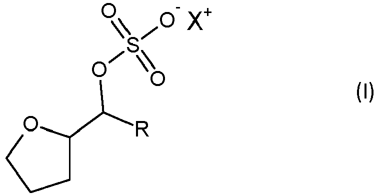 in der R für eine lineare oder verzweigte Alkyl-, Alkenyl-, Alkylaryl- oder Alkenylarylgruppe mit 5 bis 25 C-Atomen und X⁺ für ein ladungsausgleichendes Kation steht,
durch a) Umsetzung von Furfural mit einer Grignard-Verbindung R-Mg-Hal, in der R die oben angegebene Bedeutung hat und Hal ausgewählt wird aus Br und J, b) Hydrierung des Furanrings zum Tetrahydrofuranring, und c) Sulfatierung mit einem Sulfatierungsagens und gegebenenfalls Neutralisation durch anschließende Umsetzung mit X⁺OH⁻, X⁺HCO⁻₃ oderX⁺₂CO²⁻₃, wobei X⁺ für ein Alkalimetallkation oder eine Gruppierung N⁺R¹R²R³ steht, in der R¹, R² und R³ unabhängig voneinander für Wasserstoff, eine Alkylgruppe mit 1 bis 6 C-Atomen oder eine Hydroxyalkylgruppe mit 2 bis 6 C-Atomen stehen.

3. Verfahren zur Herstellung eines anionischen Tensids der allgemeinen Formel (I), 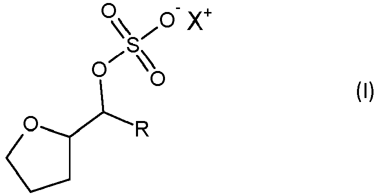 in der R für eine lineare oder verzweigte Alkyl-, Alkenyl-, Alkylaryl- oder Alkenylarylgruppe mit 5 bis 25 C-Atomen und X⁺ für ein ladungsausgleichendes Kation steht,
durch a) Umsetzung von Tetrahydrofurfural mit einer Grignard-Verbindung R-Mg-Hal, in der R die oben angegebene Bedeutung hat und Hal ausgewählt wird aus Br und J, und b) Sulfatierung mit einem Sulfatierungsagens und gegebenenfalls Neutralisation durch anschließende Umsetzung mit X⁺OH⁻, X⁺HCO⁻₃ oderX⁺₂CO²⁻₃, wobei X⁺ für ein Alkalimetallkation oder eine Gruppierung N⁺R¹R²R³ steht, in der R¹, R² und R³ unabhängig voneinander für Wasserstoff, eine Alkylgruppe mit 1 bis 6 C-Atomen oder eine Hydroxyalkylgruppe mit 2 bis 6 C-Atomen stehen.

4. Wasch- oder Reinigungsmittel, enthaltend ein anionisches Tensid der allgemeinen Formel (I), 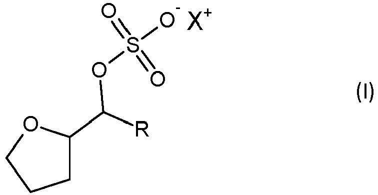 in der R für eine lineare oder verzweigte Alkyl-, Alkenyl-, Alkylaryl- oder Alkenylarylgruppe mit 5 bis 25 C-Atomen und X⁺ für ein ladungsausgleichendes Kation steht.

5. Mittel nach Anspruch 4, **dadurch gekennzeichnet, dass** es 1 Gew.-% bis 99 Gew.-%, insbesondere 3 Gew.-% bis 65 Gew.-%, des Tensids der allgemeinen Formel (I) enthält.

6. Mittel nach Anspruch 4 oder 5, **dadurch gekennzeichnet, dass** es zusätzlich bis zu 99 Gew.-%, insbesondere 3 Gew.-% bis 65 Gew.-%, weiteres Tensid enthält.

7. Mittel nach einem der Ansprüche 4 bis 6, **dadurch gekennzeichnet, dass** es teilchenförmig ist und Builder, insbesondere in einer Menge im Bereich von 1 Gew.-% bis 60 Gew.-%, enthält, oder dass es flüssig ist und 1 Gew.-% bis 90 Gew.-%, insbesondere 10 Gew.-% bis 85 Gew.-% Wasser, wassermischbares Lösungsmittel oder eines Gemisches aus Wasser und wassermischbarem Lösungsmittel enthält.

8. Mittel nach einem der Ansprüche 4 bis 6, **dadurch gekennzeichnet, dass** es einzeldosierfertig portioniert in einer aus wasserlöslichem Material gebildeten Kammer vorliegt und weniger als 15 Gew.-%, insbesondere im Bereich von 1 Gew.-% bis 12 Gew.-% Wasser enthält.

9. Verwendung eines anionischen Tensids der allgemeinen Formel (I), 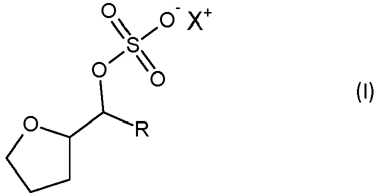 in der R für eine lineare oder verzweigte Alkyl-, Alkenyl-, Alkylaryl- oder Alkenylarylgruppe mit 5 bis 25 C-Atomen und X⁺ für ein ladungsausgleichendes Kation steht, zur Erhöhung der Leistung von Wasch- oder Reinigungsmitteln beim Waschen von Wäsche oder der Reinigung harter Oberflächen.

10. Tensid nach Anspruch 1, **dadurch gekennzeichnet, dass** in den Verbindungen der allgemeinen Formel (I) R für eine lineare oder verzweigte, insbesondere lineare, Alkylgruppe mit 7-21 C-Atomen steht.

11. Verfahren nach Anspruch 2 oder 3, **dadurch gekennzeichnet, dass** in den Verbindungen der allgemeinen Formel (I) R für eine lineare oder verzweigte, insbesondere lineare, Alkylgruppe mit 7-21 C-Atomen steht.

12. Mittel nach einem der Ansprüche 4 bis 8, **dadurch gekennzeichnet, dass** in den Verbindungen der allgemeinen Formel (I) R für eine lineare oder verzweigte, insbesondere lineare, Alkylgruppe mit 7-21 C-Atomen steht.

13. Verwendung nach Anspruch 9, **dadurch gekennzeichnet, dass** in den Verbindungen der allgemeinen Formel (I) R für eine lineare oder verzweigte, insbesondere lineare, Alkylgruppe mit 7-21 C-Atomen steht.

## Claims

1. An anionic surfactant of general formula (I), 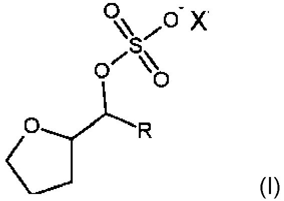 in which R represents a linear or branched alkyl, alkenyl, alkylaryl or alkenylaryl group having 5 to 25 C atoms and X⁺ represents a charge-balancing cation.

2. A method for preparing an anionic surfactant of general formula (I), 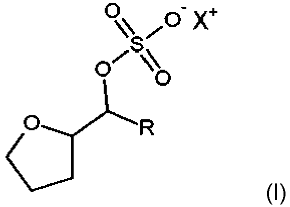 in which R represents a linear or branched alkyl, alkenyl, alkylaryl or alkenylaryl group having 5 to 25 C atoms and X⁺ represents a charge-balancing cation,
by a) reaction of furfural with a Grignard compound R-Mg-Hal, in which R has the meaning specified above and Hal is selected from Br and J, b) hydrogenation of the furan ring to form the tetrahydrofuran ring, and c) sulfation using a sulfating agent and optionally neutralization by subsequent reaction with X⁺OH⁻, X⁺HCO⁻₃ or X⁺₂CO²⁻₃, in which X⁺ represents an alkali metal cation or a grouping N⁺R¹R²R³ in which R¹, R² and R³ represent, independently of one another, hydrogen, an alkyl group having 1 to 6 C atoms or a hydroxyalkyl group having 2 to 6 C atoms.

3. A method for preparing an anionic surfactant of general formula (I), 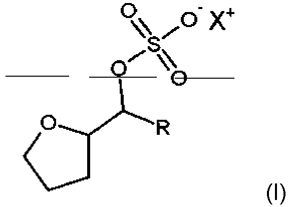 in which R represents a linear or branched alkyl, alkenyl, alkylaryl or alkenylaryl group having 5 to 25 C atoms and X⁺ represents a charge-balancing cation,
by a) reaction of tetrahydrofurfural with a Grignard compound R-Mg-Hal, in which R has the meaning specified above and Hal is selected from Br and J, and b) sulfation using a sulfating agent and optionally neutralization by subsequent reaction with X⁺OH⁻, X⁺HCO⁻₃ or X⁺₂CO²⁻₃, in which X⁺ represents an alkali metal cation or a grouping N⁺R¹R²R³ in which R¹, R² and R³ represent, independently of one another, hydrogen, an alkyl group having 1 to 6 C atoms or a hydroxyalkyl group having 2 to 6 C atoms.

4. A washing or cleaning agent containing an anionic surfactant of general formula (I), 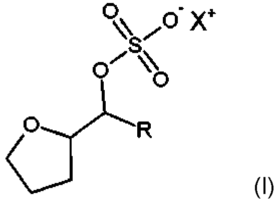 in which R represents a linear or branched alkyl, alkenyl, alkylaryl or alkenylaryl group having 5 to 25 C atoms and X⁺ represents a charge-balancing cation.

5. The agent according to claim 4, **characterized in that** it contains 1 wt.% to 99 wt.%, in particular 3 wt.% to 65 wt.%, of the surfactant of general formula (I).

6. The agent according to claim 4 or 5, **characterized in that** it additionally contains up to 99 wt.%, in particular 3 wt.% to 65 wt.%, of additional surfactant.

7. The agent according to one of claims 4 to 6, **characterized in that** it is particulate and contains builders, in particular in an amount in the range of 1 wt.% to 60 wt.%, or **in that** it is liquid and contains 1 wt.% to 90 wt.%, in particular 10 wt.% to 85 wt.%, of water, water-miscible solvent or a mixture of water and water-miscible solvent.

8. The agent according to one of claims 4 to 6, **characterized in that** it is portioned, so as to be ready for individual dosing, in a chamber formed of water-soluble material, and contains less than 15 wt.%, in particular in the range of 1 wt.% to 12 wt.%, of water.

9. The use of an anionic surfactant of general formula (I), 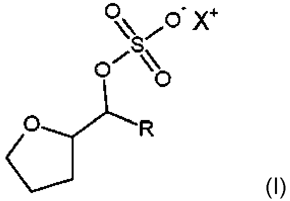 in which R represents a linear or branched alkyl, alkenyl, alkylaryl or alkenylaryl group having 5 to 25 carbon atoms and X⁺ represents a charge-balancing cation, for increasing the performance of washing or cleaning agents when washing laundry or cleaning hard surfaces.

10. The surfactant according to claim 1, **characterized in that**, in the compounds of general formula (I), R represents a linear or branched, in particular linear, alkyl group having 7-21 C atoms.

11. The method according to claim 2 or 3, **characterized in that**, in the compounds of general formula (I), R represents a linear or branched, in particular linear, alkyl group having 7-21 C atoms.

12. The agent according to one of claims 4 to 8, **characterized in that**, in the compounds of general formula (I), R represents a linear or branched, in particular linear, alkyl group having 7-21 C atoms.

13. The use according to claim 9, **characterized in that**, in the compounds of general formula (I), R represents a linear or branched, in particular linear, alkyl group having 7-21 C atoms.

## Revendications

1. Tensioactif anionique de formule générale (I), 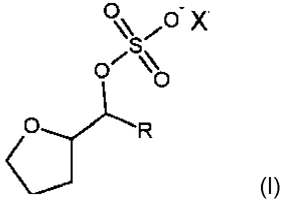 où R représente un groupe alkyle, alcényle, alkylaryle ou alcénylaryle linéaire ou ramifié ayant 5 à 25 atomes de carbone et X⁺ représente un cation compensateur de charge.

2. Procédé de préparation d'un tensioactif anionique de formule générale (I), 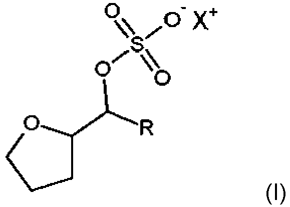 où R représente un groupe alkyle, alcényle, alkylaryle ou alcénylaryle linéaire ou ramifié ayant 5 à 25 atomes de carbone et X⁺ représente un cation compensateur de charge,
par a) réaction de furfural avec un composé de Grignard R-Mg-Hal, où R présente la signification donnée ci-dessus et Hal est choisi entre Br et J, b) hydrogénation du cycle furanique en cycle tétrahydrofuranique, et c) sulfatation avec un agent de sulfatation et éventuellement neutralisation par réaction ultérieure avec X⁺OH⁻, X⁺HCO⁻₃ ou X⁺₂CO²⁻₃, où X⁺ représente un cation de métal alcalin ou un groupement N⁺R¹R²R³, où R¹, R² et R³ représentent indépendamment les uns des autres l'hydrogène, un groupe alkyle ayant 1 à 6 atomes de carbone ou un groupe hydroxyalkyle ayant 2 à 6 atomes de carbone.

3. Procédé de préparation d'un tensioactif anionique de formule générale (I), 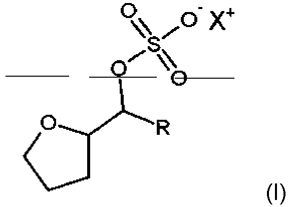 où R représente un groupe alkyle, alcényle, alkylaryle ou alcénylaryle linéaire ou ramifié ayant 5 à 25 atomes de carbone et X⁺ représente un cation compensateur de charge,
par a) réaction de tétrahydrofurfural avec un composé de Grignard R-Mg-Hal, où R présente la signification donnée ci-dessus et Hal est choisi entre Br et J, et b) sulfatation avec un agent de sulfatation et éventuellement neutralisation par réaction ultérieure avec X⁺OH⁻, X⁺HCO⁻₃ ou X⁺₂CO²⁻₃, où X⁺ représente un cation de métal alcalin ou un groupement N⁺R¹R²R³, où R¹, R² et R³ représentent indépendamment les uns des autres l'hydrogène, un groupe alkyle ayant 1 à 6 atomes de carbone ou un groupe hydroxyalkyle ayant 2 à 6 atomes de carbone.

4. Agent de lavage ou de nettoyage, contenant un tensioactif anionique de formule générale (I), 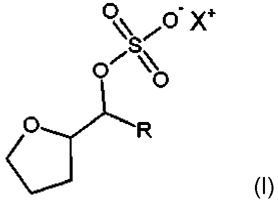 où R représente un groupe alkyle, alcényle, alkylaryle ou alcénylaryle linéaire ou ramifié ayant 5 à 25 atomes de carbone et X⁺ représente un cation compensateur de charge.

5. Agent selon la revendication 4, **caractérisé en ce qu'**il contient 1 % en poids à 99 % en poids, en particulier 3 % en poids à 65 % en poids, du tensioactif de formule générale (I).

6. Agent selon la revendication 4 ou 5, **caractérisé en ce qu'**il contient en outre jusqu'à 99 % en poids, en particulier de 3 % en poids à 65 % en poids, d'un autre tensioactif.

7. Agent selon l'une quelconque des revendications 4 à 6, **caractérisé en ce qu'**il est sous forme particulaire et contient des adjuvants, en particulier en une quantité dans la plage comprise entre 1 % en poids et 60 % en poids, ou qu'il est liquide et contient 1 % en poids à 90 % en poids, en particulier 10 % en poids à 85 % en poids, d'eau, de solvant miscible dans l'eau ou d'un mélange d'eau et de solvant miscible dans l'eau.

8. Agent selon l'une quelconque des revendications 4 à 6, **caractérisé en ce qu'**il est présent sous forme de portions individuelles prêtes à l'emploi dans une chambre formée à partir d'une matière hydrosoluble et contient moins de 15 % en poids, en particulier dans la plage comprise entre 1 % en poids et 12 % en poids, d'eau.

9. Utilisation d'un tensioactif anionique de formule générale (I), 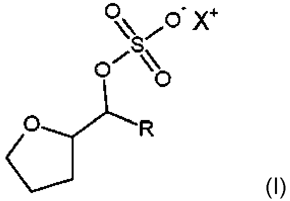 où R représente un groupe alkyle, alcényle, alkylaryle ou alcénylaryle linéaire ou ramifié ayant 5 à 25 atomes de carbone et X⁺ représente un cation compensateur de charge, pour l'augmentation des performances d'agents de lavage ou de nettoyage lors du lavage du linge ou du nettoyage des surfaces dures.

10. Tensioactif selon la revendication 1, **caractérisé en ce que** dans les composés de formule générale (I), R représente un groupe alkyle linéaire ou ramifié, en particulier linéaire, ayant 7 à 21 atomes de carbone.

11. Procédé selon la revendication 2 ou 3, **caractérisé en ce que** dans les composés de formule générale (I), R représente un groupe alkyle linéaire ou ramifié, en particulier linéaire, ayant 7 à 21 atomes de carbone.

12. Agent selon l'une quelconque des revendications 4 à 8, **caractérisé en ce que** dans les composés de formule générale (I), R représente un groupe alkyle linéaire ou ramifié, en particulier linéaire, ayant 7 à 21 atomes de carbone.

13. Utilisation selon la revendication 9, **caractérisée en ce que** dans les composés de formule générale (I), R représente un groupe alkyle linéaire ou ramifié, en particulier linéaire, ayant 7 à 21 atomes de carbone.
